(19) Europäisches Patentamt
European Patent Office
Office européen des brevets

(11) **EP 4 454 688 A1**

(12) **EUROPEAN PATENT APPLICATION**
published in accordance with Art. 153(4) EPC

(43) Date of publication:
**30.10.2024 Bulletin 2024/44**

(21) Application number: **21968664.9**

(22) Date of filing: **24.12.2021**

(51) International Patent Classification (IPC):
**A61M 16/00** $^{(2006.01)}$

(52) Cooperative Patent Classification (CPC):
**A61M 16/00**

(86) International application number:
**PCT/CN2021/141168**

(87) International publication number:
**WO 2023/115531 (29.06.2023 Gazette 2023/26)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Shenzhen Mindray Bio-Medical Electronics Co., Ltd.**
**Shenzhen, Guangdong 518057 (CN)**

(72) Inventors:
• ZOU, Xinru
Shenzhen, Guangdong 518057 (CN)
• LIU, Jinglei
Shenzhen, Guangdong 518057 (CN)
• ZHOU, Xiaoyong
Shenzhen, Guangdong 518057 (CN)

(74) Representative: **KIPA AB**
**Drottninggatan 11**
**252 21 Helsingborg (SE)**

(54) **RESPIRATION MONITORING METHOD AND RESPIRATION MONITORING APPARATUS**

(57) A respiration monitoring method and a respiration monitoring apparatus. The method comprises: for airway pressure data collected by a pressure sensor and gas flow rate data measured by a flow sensor within one respiratory cycle, calculating the intrapulmonary pressure of a patient in an inspiratory phase and/or an expiratory pressure value of the patient, which intrapulmonary pressure and expiratory pressure value correspond to the respiration cycle; and according to the intrapulmonary pressure of the inspiratory phase and the expiratory pressure value, determining a driving pressure of the patient that corresponds to the respiration cycle. In this way, a driving pressure can be obtained without manual intervention such as inspiratory and expiratory hold, and the driving pressure can also be measured in real time; and since ventilation is not interfered with, a patient-friendly effect is also achieved.

```
┌─────────────────────────────────────────────┐
│ Calculate, in real time, end-inspiratory     │     1
│ intrapulmonary pressure and end-expiratory    │
│ intrapulmonary pressure, according to         │
│ respiratory cycle                             │
└─────────────────────────────────────────────┘
                      │
┌─────────────────────────────────────────────┐
│ Subtract end-expiratory intrapulmonary        │     2
│ pressure from end-inspiratory intrapulmonary  │
│ pressure to obtain real-time driving pressure │
└─────────────────────────────────────────────┘
```

FIG. 4

**Description**

TECHNICAL FIELD

The disclosure relates to a medical field, and more particularly to a respiration monitoring method and a respiration monitoring apparatus.

BACKGROUND

During mechanical ventilation, unreasonable settings of a ventilator can cause lung damage to a patient. In 2000, a multicentre study of the UK ARDSnet proposed a ventilation strategy for lung protection with a small tidal volume (VT 6-8 ml/kg ideal body weight), a low airway plateau pressure (Pplat <30-35 cmH2O), a high positive end-expiratory pressure (PEEP), and permissive hypercapnia (PHC) as cores. At present, the ventilation strategy for lung protection prioritizes maintaining a low tidal volume. However, clinical evidence shows that a mortality rate of ARDS does not decrease with ventilation of low tidal volume [1]. This is because ARDS patients have different causes, different severities, different functional residual capacities, different abilities of lung recruitment, different sizes and distributions of collapsed alveoli, and these differences result in lung heterogeneity, which makes lung compliance different for different ARDS patients. Accordingly, tidal volumes, which are actually required by the patients, are also different. Therefore, it is not enough to use the tidal volume alone as a lung protection control. The commonly used ventilation strategy for lung protection of small tidal volume combined with optimal positive end-expiratory pressure (PEEP) still causes over-expansion of alveoli in non-gravity-dependent areas and tidal collapse and re-expansion of alveoli in gravity-dependent areas, making ventilator-related lung injury inevitable. In addition, combined with the lung protection method of small tidal volume and plateau pressure limitation, lung damage and high pressure may still occur, because patients with many collapsed alveoli have fewer normally ventilated alveoli and over-expansion is significantly increased. For different patients having the same tidal volume but different compliance, pressures acting on the lungs are different. Therefore, it is particularly important to monitor a driving pressure, which is power that directly drives an expansion of an entire respiratory system, in combination with Pplat which avoids excessive expansion of the alveoli and PEEP which avoids alveolar collapse.

Existing methods for measuring a driving pressure is as follows.

Airway pressure is a pressure acting on the respiratory system, including an airway resistance, and pressure(s) which act(s) on lungs, and chest wall. Intrapulmonary pressure is a pressure which acts directly on the lungs and chest wall after removing the airway resistance. Transpulmonary pressure is a pressure which acts directly on the lungs, which is a result of removing a part of the intrapulmonary pressure, which part acts on the chest wall. The transpulmonary pressure is obtained by subtracting an intrathoracic pressure from the intrapulmonary pressure. The intrathoracic pressure is usually approximated with an esophageal pressure, that is, transpulmonary pressure = intrapulmonary pressure - esophageal pressure.

Driving pressure is routinely measured clinically as follows. The plateau pressure is measured by inspiratory hold, and PEEPtotal (PEEPi+PEEP) is measured by expiratory hold. Driving pressure = plateau pressure measured during inspiratory hold- PEEPtotal (PEEPi+PEEP) measured during expiratory hold. The purposes of inspiratory hold and expiratory hold are making the airway pressure equal to the intrapulmonary pressure after an airflow inside the airway stabilizes, removing an interference of airway resistance, and obtaining a real pressure inside the lungs. The driving pressure reflects a change in the intrapulmonary pressure after removing an effect of airway resistance.

In clinical practice, when chest wall compliance of some obese patients or patients with abdominal hypertension is low, the intrapulmonary pressure is mainly distributed on the chest wall, and the pressure which acts on the lungs is very small. Therefore, for such patients, the intrapulmonary pressure cannot reflect the actual pressure which acts on the lungs, and transpulmonary pressure (the pressure which acts directly on the lungs after removing the part of pressure acting on the chest wall) is needed for monitoring. Transpulmonary driving pressure = transpulmonary pressure measured during inspiratory hold - transpulmonary pressure measured during expiratory hold.

In the current methods for measuring the driving pressure, operations of inspiratory hold and expiratory hold are relatively complicated, which require recording data before calculation, and interfere with ventilation.

SUMMARY

This disclosure mainly provides a respiration monitoring method and a respiration monitoring apparatus, which is capable of obtaining a driving pressure without requiring expiratory hold and inspiratory hold.

In an embodiment, a respiration monitoring apparatus is provided, including:

a pressure sensor, which is configured to obtain an airway pressure of a patient, or obtain an airway pressure and an esophageal pressure of a patient, so as to obtain corresponding airway pressure data, or obtain corresponding airway pressure data and esophageal pressure data;
a flow sensor, which is configured to obtain gas flow rate data of a patient during a ventilation process;
a processor, which is configured to:

according to airway pressure data which are measured by the pressure sensor in a respiratory cycle and gas flow rate data which are measured by the flow sensor in said respiratory cycle, calculate an intrapulmonary pressure of an inspiratory phase of a patient and/or an expiratory pressure of said patient, which pressures correspond to said respiratory cycle; and determine a driving pressure of said patient, which pressure corresponds to said respiratory cycle, according to the intrapulmonary pressure of the inspiratory phase and the expiratory pressure; or

according to airway pressure data which are measured by the pressure sensor in a respiratory cycle and gas flow rate data which are measured by the flow sensor in said respiratory cycle, calculate an intrapulmonary pressure of an inspiratory phase of a patient and an intrapulmonary pressure of an expiratory phase of said patient, which pressures correspond to said respiratory cycle; and determine a transpulmonary driving pressure of said patient, which pressure corresponds to said respiratory cycle, according to the intrapulmonary pressure of the inspiratory phase and the intrapulmonary pressure of the expiratory phase, as well as esophageal pressure data which are measured by the pressure sensor in said respiratory cycle.

In a respiration monitoring apparatus provided in an embodiment, in order to determine the transpulmonary driving pressure of said patient, the processor is further configured to:

according to the airway pressure data which are measured by the pressure sensor in said respiratory cycle and the gas flow rate data which are measured by the flow sensor in said respiratory cycle, calculate an end-inspiratory intrapulmonary pressure of said patient and an end-expiratory intrapulmonary pressure of said patient, which pressures correspond to said respiratory cycle; and determine the transpulmonary driving pressure of said patient, which pressure corresponds to said respiratory cycle, according to the end-inspiratory intrapulmonary pressure and the end-expiratory pulmonary pressure, as well as the esophageal pressure data which are measured by the pressure sensor in said respiratory cycle; or

according to the airway pressure data which are measured by the pressure sensor in said respiratory cycle and the gas flow rate data which are measured by the flow sensor in said respiratory cycle, calculate an intrapulmonary pressure curve for the inspiratory phase of said patient and an intrapulmonary pressure curve for the expiratory phase of said patient, which curves correspond to said respiratory cycle; and determine a transpulmonary pressure curve which corresponds to said respiratory cycle, according to the intrapulmonary pressure curve for the inspiratory phase and the intrapulmonary pressure curve for the expiratory phase, as well as an esophageal pressure curve which is measured by the pressure sensor in said respiratory cycle; determine an end-inspiratory transpulmonary pressure and an end-expiratory transpulmonary pressure according to the transpulmonary pressure curve, so as to determine the transpulmonary driving pressure of said patient, which pressure corresponds to said respiratory cycle.

In a respiration monitoring apparatus provided in an embodiment, a display apparatus is further included, wherein the processor is further configured to transmit the driving pressure or the transpulmonary driving pressure to the display apparatus for displaying.

In a respiration monitoring apparatus provided in an embodiment, the driving pressure or the transpulmonary driving pressure is displayed on the display apparatus in a graph which changes with time.

In a respiration monitoring apparatus provided in an embodiment, the intrapulmonary pressure of the inspiratory phase includes the end-inspiratory intrapulmonary pressure, the intrapulmonary pressure of the expiratory phase includes the end-expiratory intrapulmonary pressure;

in order to calculate the end-inspiratory intrapulmonary pressure and the end-expiratory intrapulmonary pressure, the processor is further configured to:

obtain, in real time, the gas flow rate data which are measured by the flow sensor during a ventilation process of said respiratory cycle of said patient, and the airway pressure data which are measured by the pressure sensor during the ventilation process of said respiratory cycle of said patient;
calculate intrapulmonary pressure data, which correspond to said respiratory cycle, according to the gas flow rate data and the airway pressure data which correspond to said respiratory cycle; wherein the intrapulmonary pressure data include the end-inspiratory intrapulmonary pressure and the end-expiratory intrapulmonary pressure.

In a respiration monitoring apparatus provided in an embodiment, in order to determine the transpulmonary driving pressure of said patient, the processor is further configured to:

for said respiratory cycle, calculate intrapulmonary pressure data at an inspiratory end of said respiratory cycle, and intrapulmonary pressure data at an expiratory end of said respiratory cycle, according to the gas flow rate data and the airway pressure data which correspond to said respiratory cycle;

obtain transpulmonary pressure data which respectively correspond to the inspiratory end of said respiratory cycle and the expiratory end of said respiratory cycle, according to difference(s) between the intrapulmonary pressure data at the inspiratory end and esophageal pressure data which are measured by the pressure senor at corresponding time point(s), and difference(s) between the intrapulmonary pressure data at the expiratory end and esophageal pressure data which are measured by the pressure senor at corresponding time point(s); wherein the intrapulmonary pressure data includes an end-inspiratory intrapulmonary pressure and an end-expiratory intrapulmonary pressure;

obtain the transpulmonary driving pressure which corresponds to said respiratory cycle, according to the end-inspiratory transpulmonary pressure and the end-expiratory transpulmonary pressure.

In a respiration monitoring apparatus provided in an embodiment, the intrapulmonary pressure data includes waveform(s) for intrapulmonary pressure(s), the esophageal pressure data includes waveform(s) for esophageal pressure(s) , and the transpulmonary pressure data includes waveform(s) for transpulmonary pressure(s); or

the intrapulmonary pressure data includes an end-inspiratory intrapulmonary pressure and an end-expiratory intrapulmonary pressure, and the esophageal pressure data include an end-inspiratory esophageal pressure and an end-expiratory esophageal pressure;

in order to calculate differences between the intrapulmonary pressure data and the esophageal pressure data, so as to obtain the intrapulmonary pressure data which correspond to said respiratory cycle, the processor is further configured to:

subtract the end-inspiratory esophageal pressure from the end-inspiratory intrapulmonary pressure to obtain a corresponding end-inspiratory transpulmonary pressure; and

subtract the end-expiratory esophageal pressure from the end-expiratory intrapulmonary pressure to obtain a corresponding end-expiratory transpulmonary pressure.

In a respiration monitoring apparatus provided in an embodiment, the gas flow rate data include a gas flow rate which corresponds to the inspiratory phase of said respiratory cycle, and a gas flow rate which corresponds to the expiratory phase of said respiratory cycle; the airway pressure data include an airway pressure which corresponds to the inspiratory phase of said respiratory cycle, and an airway pressure which corresponds to the expiratory phase of said respiratory cycle;

in order to calculate intrapulmonary pressure data, which correspond to said respiratory cycle, according to the gas flow rate data, and the airway pressure data which correspond to said respiratory cycle, the processor is further configured to:

calculate a gas volume which corresponds to the inspiratory phase, according to the gas flow rate which corresponds to the inspiratory phase;

calculate the intrapulmonary pressure of the inspiratory phase, according to the airway pressure which corresponds to the inspiratory phase, the gas volume which corresponds to the inspiratory phase and the gas flow rate which corresponds to the inspiratory phase;

calculate the intrapulmonary pressure of the expiratory phase, according to the airway pressure which corresponds to the expiratory phase, and the gas flow rate which corresponds to the expiratory phase; and

obtain waveform(s) for intrapulmonary pressure(s) of said patient in said respiratory cycle according to the intrapulmonary pressure of the inspiratory phase and the intrapulmonary pressure of the expiratory phase;

or

the airway pressure data include an end-inspiratory airway pressure and an end-expiratory airway pressure;

in order to calculate intrapulmonary pressure data, which correspond to said respiratory cycle, according to the gas flow rate data, and the airway pressure data which correspond to said respiratory cycle, the processor is further configured to:

calculate a total gas volume which corresponds to the inspiratory phase, according to the gas flow rate data;

calculate the end-inspiratory intrapulmonary pressure according to the end-inspiratory airway pressure, the total gas volume which corresponds to the inspiratory phase, an end-inspiratory flow rate in the gas flow rate data, and the end-expiratory airway pressure;

calculate the end-expiratory intrapulmonary pressure according to the end-expiratory airway pressure and an end-expiratory flow rate in the gas flow rate data, or approximate the end-expiratory intrapulmonary pressure with the end-expiratory airway pressure.

In a respiration monitoring apparatus provided in an embodiment, the processor is further configured to:

obtain, through the flow sensor in real time, a tidal volume of said patient;
obtain corresponding compliance according to the tidal volume and the driving pressure, or obtain corresponding lung compliance according to the tidal volume and the transpulmonary driving pressure; and
monitor a respiratory state of said patient according to the compliance or the lung compliance.

In a respiration monitoring apparatus provided in an embodiment, the processor is further configured to:

analyse the driving pressure or the transpulmonary driving pressure according to a preset condition; and
transmit an indication signal, when the driving pressure or the transpulmonary driving pressure does not satisfy the preset condition.

In one embodiment, a respiration monitoring method is provided, including:

during a ventilation process in real time, obtaining airway pressure data and gas flow rate data of a patient, or obtaining airway pressure data, esophageal pressure data and gas flow rate data of a patient;
for a respiratory cycle, calculating corresponding intrapulmonary pressure data according to gas flow rate data and airway pressure data in said respiratory cycle, wherein the intrapulmonary pressure data include an end-inspiratory intrapulmonary pressure and an end-expiratory intrapulmonary pressure; the end-inspiratory intrapulmonary pressure and the end-expiratory intrapulmonary pressure respectively characterize a pressure which acts on an expected position of a respiratory system of said patient at an inspiratory end and an expiratory end respectively during the ventilation process; and obtaining a driving pressure which corresponds to the respiratory cycle according to the end-inspiratory intrapulmonary pressure and the end-expiratory intrapulmonary pressure; or
according to airway pressure data which are measured by a pressure sensor in a respiratory cycle and gas flow rate data which are measured by a flow sensor in said respiratory cycle, calculating an intrapulmonary pressure of an inspiratory phase of a patient and an intrapulmonary pressure of an expiratory phase of said patient, which pressures correspond to said respiratory cycle; and determining a transpulmonary driving pressure of said patient, which pressure corresponds to said respiratory cycle, according to the intrapulmonary pressure of the inspiratory phase and the intrapulmonary pressure of the expiratory phase, as well as esophageal pressure data which are measured by the pressure sensor in said respiratory cycle.

In one embodiment, a computer-readable storage medium is provided, on which program(s) is(are) stored, wherein the program(s) is(are) capable of being executed, so as to implement the respiration monitoring method discussed above.

According to a respiration monitoring method and a respiration monitoring apparatus of above-mentioned embodiment(s), an intrapulmonary pressure of an inspiratory phase of a patient and/or an expiratory pressure of said patient, which pressure(s) correspond(s) to a respiratory cycle, is/are capable of being calculated according to airway pressure data which are measured by the pressure sensor in the respiratory cycle and the gas flow rate data which are measured by the flow sensor in the respiratory cycle, and a driving pressure of the patient, which pressure corresponds to the respiratory cycle, is capable of being determined according to an intrapulmonary pressure of the inspiratory phase and an expiratory pressure, which pressures correspond to the respiratory cycle. In this way, the driving pressure can be obtained without manual intervention, such as inspiratory hold and expiratory hold, and the driving pressure can be detected in real time without interfering with ventilation while being patient-friendly.

BRIEF DESCRIPTION OF THE DRAWINGS

FIG. 1 is a structural diagram of an embodiment of a respiration monitoring apparatus provided by this disclosure.
FIG. 2 is a structural diagram of another embodiment of a respiration monitoring apparatus provided by this disclosure.
FIG. 3 is a structural diagram of an embodiment of a medical ventilation device in a respiration monitoring apparatus provided by this disclosure.
FIG. 4 is a flow chart of an embodiment of a respiration monitoring method provided by this disclosure.
FIG. 5 is a flow chart of an embodiment of step 1 in FIG. 4 .
FIG. 6 is a flow chart of another embodiment of step 1 in FIG. 4 .
FIG. 7 is a schematic diagram of a respiratory mechanics RC model.
FIG. 8 is a waveform diagram of an airway pressure and an intrapulmonary pressure in a respiration monitoring apparatus provided by this disclosure.

FIG. 9 is a flow chart of another embodiment of a respiration monitoring method provided by this disclosure.

DESCRIPTION OF THE PREFERRED EMBODIMENTS

This disclosure is further described in detail below through specific embodiments in combination with the accompanying drawings. In different embodiments, similar elements adopt associated similar reference numbers. In the following embodiments, many details are described in order to make this disclosure better understood. However, one skilled in the art can easily recognize that some of the features can be omitted in different cases, or can be replaced by other elements, materials and methods. In some cases, some operations related to this disclosure are not shown or described in the description, in order to avoid the core part of this disclosure being inundated by too many descriptions. For one skilled in the art, it is not necessary to describe these related operations in detail, as they can fully understand the relevant operations according to the description in this disclosure and the general technical knowledge in the art.

In addition, the features, operations, or characteristics described in the description may be combined in any appropriate manner to form various embodiments. At the same time, the steps or actions in the method description can also be exchanged or adjusted sequentially in a manner obvious to one skilled in the art. Therefore, the various sequences in the description and the drawings are only for the purpose of clearly describing a certain embodiment, and do not mean that they are necessary sequences. Unless it is otherwise specified that one of the sequences must be followed.

The terms "first", "second", etc. in the description are operable to distinguish different objects, rather than to describe a specific order. In addition, the terms "connection", and "linkage" mentioned in this disclosure include direct and indirect connection (linkage), unless otherwise specified.

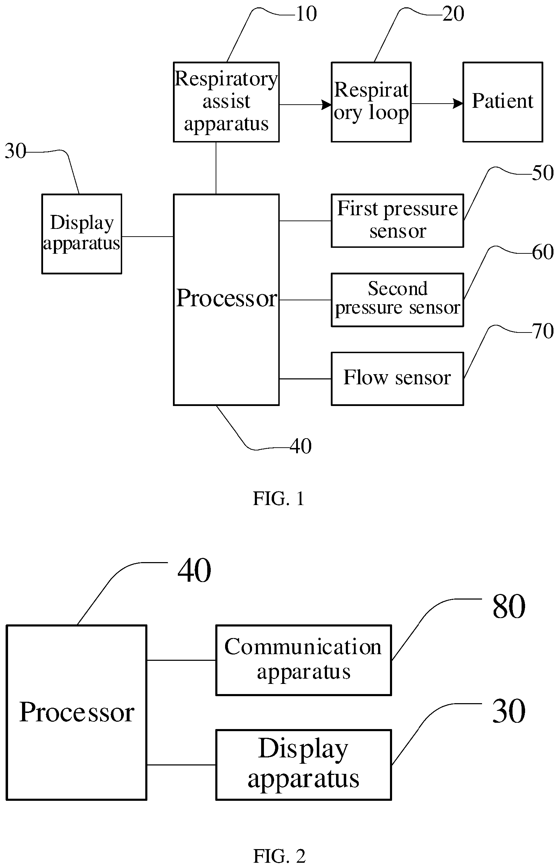

Referring FIGS. 1-2, some embodiments of this disclosure disclose a respiration monitoring apparatus, including a processor 40 and a display apparatus 30.

The processor 40 is configured to obtain at least one of airway pressure data and esophageal pressure data of a patient. That is, the processor 40 is capable of obtaining airway pressure data, or esophageal pressure data, or both airway pressure data and esophageal pressure data. The processor 40 is further configured to obtain gas flow rate data of a patient during a ventilation process.

The processor 40 is configured to calculate an intrapulmonary pressure of an inspiratory phase and/or an expiratory pressure, of a patient, based on airway pressure data and gas flow rate data, according to a respiratory cycle; to determine and output a driving pressure of the patient based on the intrapulmonary pressure of the inspiratory phase and the expiratory pressure, such as to subtract the expiratory pressure from the intrapulmonary pressure of the inspiratory phase, so as to obtain the driving pressure of the patient. The expiratory pressure may be a positive end-expiratory pressure directly measured by a ventilator through a sensor, or may be an intrapulmonary pressure of an expiratory phase which is calculated based on the airway pressure data and the gas flow rate data.

The processor 40 is further configured to calculate an intrapulmonary pressure of an inspiratory phase and an intrapulmonary pressure of an expiratory phase, of the patient, based on the airway pressure data and the gas flow rate data, according to the respiratory cycle; to determine and output a transpulmonary driving pressure of the patient, based on the intrapulmonary pressure of the inspiratory phase and the intrapulmonary pressure of the expiratory phase, as well as an esophageal pressure.

When the processor 40 outputs the driving pressure and/or the transpulmonary driving pressure, the processor 40 may output the pressure(s) to an external device (such as a printer, a monitor, etc.), or to a display apparatus 30 (such as a display), through which the driving pressure(s) of the patient is(are) displayed. For example, the processor 40 outputs to the display apparatus 30, the driving pressure of and/or the transpulmonary driving pressure, of the patient, and the display apparatus 30 receives and displays the driving pressure and/or the transpulmonary driving pressure which is(are) transmitted by the processor 40. The display apparatus 30 may display the driving pressure and/or the transpulmonary driving pressure in a variety of ways, such as directly displaying numerical value(s) of the driving pressure and/or the transpulmonary driving pressure, or displaying the driving pressure and/or the transpulmonary driving pressure in a form of a graph or chart (e.g., a bar graph), etc. The user can see approximate range(s) of the numerical values of the driving pressure and/or the transpulmonary driving pressure through the graph or chart, etc. In short, it is sufficient that, values of the driving pressure and/or the transpulmonary driving pressure can be reflected to a certain extent. The display apparatus 30 can output visual information, and may also be various types of displays.

It can be seen that this disclosure can obtain the driving pressure and/or the transpulmonary driving pressure without artificial intervention, such as inspiratory hold and expiratory hold, and can also detect the driving pressure and/or the transpulmonary driving pressure in real time without interfering with ventilation while being patient-friendly.

The respiration monitoring apparatus of this disclosure can be applied in a variety of occasions. For example, in some embodiments, the respiration monitoring apparatus of this disclosure can be a monitor, a central station, a monitoring module, etc. In some embodiments, it can be a medical ventilation device, such as a ventilator, an anesthesia machine, a portable handheld ventilation device, etc. In some embodiments, it can also be other medical devices with computing and processing capabilities. When the types of respiration monitoring apparatuses are different, and the ways in which the processor 40 obtains the above data may also be different. For example, as shown in FIG. 1, the respiration monitoring apparatus also includes a pressure sensor and a flow sensor 70, etc. The pressure sensor is configured to obtain an airway pressure of the patient to obtain corresponding airway pressure data, or to obtain an airway pressure and an esophageal pressure to obtain corresponding airway pressure data and esophageal pressure data. The flow sensor 70 is configured to measure gas flow rate data of the patient during a ventilation process. The processor 40 is configured to obtain the airway pressure data and the esophageal pressure data through the pressure sensor of the respiration monitoring apparatus itself, and to obtain the gas flow rate data through the flow sensor 70. For another example, the respiration monitoring apparatus is a monitor, as shown in FIG. 2, and further includes a communication apparatus 80, and the processor 40 is configured to obtain airway pressure data, esophageal pressure data, and gas flow rate data from other external devices, through the communication apparatus 80.

To facilitate a more detailed description of a technical solution of this disclosure, this embodiment is described by taking the respiration monitoring apparatus as a medical ventilation device for example. Medical ventilation device can perform mechanical ventilation to assist or control a spontaneous respiratory movement of a patient, so as to achieve a function of gas exchange in the lungs, reduce body consumption, and facilitate a recovery of respiratory function. As shown in FIG. 1, the medical ventilation device further includes a respiratory assist apparatus 10 and a respiratory loop 20.

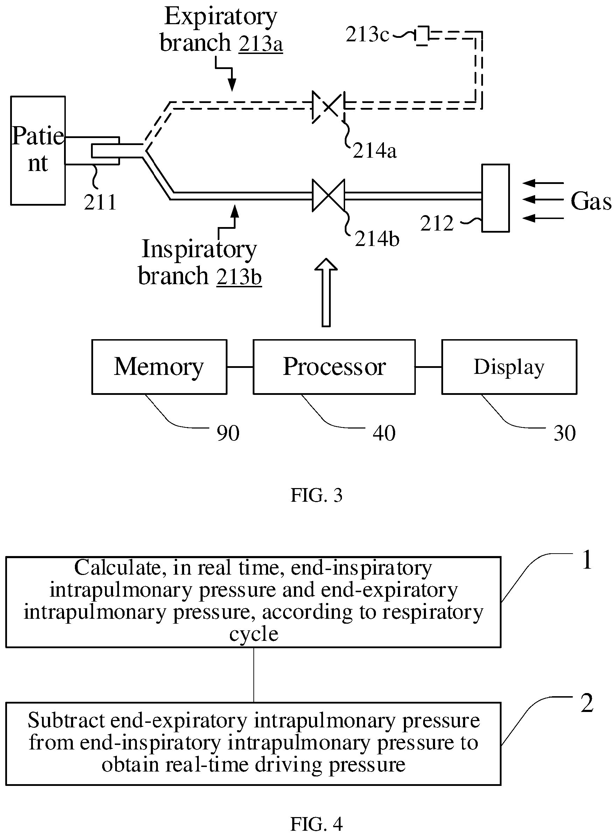

The medical ventilation device takes a ventilator as an example, as shown in FIG. 3, which shows an invasive ventilator, further including a respiratory interface 211, a gas source interface 212 and a memory 90 .

The respiratory assist apparatus 10 is configured to provide power to input a preset gas to a patient, or to discharge at least a portion of a gas exhaled by the patient to an external environment. The preset gas is a gas that satisfies a respiratory requirement of the patient. For an anesthesia machine, the preset gas may also contain anaesthetics.

The respiratory loop 20 selectively connects the gas source interface 212 and a respiratory system of the patient, and includes at least one branch (such as an expiratory branch 213a and an inspiratory branch 213b) and a valve arranged at the branch, wherein the valve is configured to open or close the branch. The respiratory loop 20 is configured to transmit the preset gas to the patient through the inspiratory branch 213b, and/or to discharge at least portion of the gas exhaled by the patient to the external environment through the expiratory branch 213a. In some embodiments, such as an invasive ventilator, the respiratory loop 20 includes an expiratory branch 213a and an inspiratory branch 213b. The expiratory branch 213a is connected between the respiratory interface 211 and an exhaust port 213c to guide the exhaled gas of the patient to an exhaust port 213c. The exhaust port 213c can be connected with the external environment or to a dedicated gas recovery apparatus. In some embodiments such as a non-invasive ventilator, the respiratory loop 20 includes an inspiratory branch 213b but does not require an expiratory branch 213a, and the patient exhales spontaneously. The gas source interface 212 is configured to connect to a gas source (not shown), which is configured to provide gas, and the gas can generally be oxygen, air, etc. In some embodiments, the gas source can be a compressed gas cylinder or a central gas supply source, and the ventilator is supplied with a gas through the gas source interface 212. The types of gas supplied include oxygen $O_2$ and air, etc. The gas source interface 212 may include conventional components, such as a pressure gauge, a pressure regulator, a flow meter, a pressure reducing valve, and a control and protection device for air-oxygen ratio, which are respectively used to control flows of various gases (such as oxygen and air). Of course, the gas source may also be a turbine, through which the gas is outputted to the gas source interface 212, or the gas source interface 212 may be directly replaced by a turbine. The inspiratory branch 213b is connected between the respiratory interface 211 and the gas source interface 212, and is configured to provide the patient with a preset gas (such as oxygen or air). For example, the gas inputted from the gas source interface 212 enters the inspiratory branch 213b, and then enters the lungs of the patient through the respiratory interface 211. The respiratory interface 211 is configured to connect the patient to the respiratory loop 20. In addition to introducing the gas transmitted by the inspiratory branch 213b to the patient, for an invasive ventilator, it can also introduce the gas exhaled by the patient to the exhaust port 213c through the expiratory branch 213a, and for a non-invasive ventilator, it can also directly exhaust the gas exhaled by the patient. Depending on situations, the respiratory interface 211 can be a nasal cannula or a mask worn on mouth and nose.

The respiratory assist apparatus 10 is connected with the gas source interface 212 and the respiratory loop 20 to control the gas provided by the external gas source to be delivered to the patient through the respiratory loop 20. In an embodiment of an invasive ventilator, the respiratory assist apparatus 10 may include an expiratory controller 214a and an inspiratory controller 214b, and in an embodiment of a non-invasive ventilator, the respiratory assist apparatus 10 may include an inspiratory controller 214b. The expiratory controller 214a is arranged at the expiratory branch 213a, and is configured to connect or disconnect the expiratory branch 213a according to a control instruction, or to control a flow rate or a pressure of the exhaled gas of the patient. In a specific implementation, the expiratory controller 214a may include one or more of devices that can control a flow rate or a pressure, such as an expiratory valve, a one-way valve, a flow controller, a PEEP valve, and others. The inspiratory controller 214b is arranged at the inspiratory branch 213b, and is configured to connect or disconnect the inspiratory branch 213b according to a control instruction, or to control a flow rate or a pressure of the output gas. In specific implementation, the inspiratory controller 214b may include one or more of devices that can control a flow rate or pressure, such as an inspiratory valve, a one-way valve, a flow controller, a PEEP valve, and others.

The memory 90 can be configured to store data or program(s), such as data obtained by a sensor, data calculated by a processor, or image frame(s) generated by a processor. The image frame(s) can be a 2D or 3D image(s), or the memory 90 can store a graphic user interface, one or more default image display settings, and programming instruction(s) for a processor. The memory 90 may be a tangible and non-transitory computer-readable medium, such as flash memory, RAM, ROM, EEPROM, etc.

In some embodiments, the processor 40 is configured to execute instruction(s) or program(s), so as to control the respiratory assistance apparatus 10, the gas source interface 212 and/or various control valves in the respiratory loop 20, or so as to process the received data to generate a required calculation or determination result, or so as to generate visual data or graphic(s) and output the visual data or graphic(s) to the display 30 for displaying.

The above are some descriptions of the medical ventilation device as a ventilator. It should be noted that FIG. 3 above is only an example of a ventilator, and it is not intended to limit the ventilator to just have such a structure.

The pressure sensor is configured to obtain at least one of an airway pressure and an esophageal pressure, of a patient, so as to obtain at least one of airway pressure data and esophageal pressure data. In this embodiment, the respiration monitoring apparatus includes a first pressure sensor 50 and a second pressure sensor 60. The first pressure sensor 50 is configured to obtain the esophageal pressure of the patient. In this embodiment, the esophageal pressure of the patient is detected in real time to obtain the esophageal pressure data. The second pressure sensor 60 is configured to obtain the airway pressure of the patient, when the medical ventilation device is ventilating the patient. In this embodiment, the airway pressure of the patient is detected in real time to obtain the airway pressure data.

The flow sensor 70 is configured to obtain a gas flow rate of the patient during a ventilation process. In this embodiment, the gas flow rate of the patient during the ventilation process is detected in real time to obtain the gas flow rate data. The flow sensor 70 may be arranged at a gas path between the respiratory interface 211 and the gas source interface 212, or a gas path between the respiratory interface 211 and the exhaust port 213c. The gas flow rate can be a flow rate of the gas, which gas enters the body of the patient. For invasive respiration, in some embodiments, multiple flow sensors 70 exist, and include an inspiratory flow sensor and an expiratory flow sensor arranged at a mechanical ventilation end. For example, for a ventilator, the multiple flow sensors 70 can include an inspiratory flow sensor arranged inside the inspiratory branch 213b, and an expiratory flow sensor arranged inside the expiratory branch 213a. In some embodiments, the flow sensor 70 can also be a Y-piece flow sensor, which is directly connected with a patient end, and the flow rate, which flows into and out of the patient end, is directly measured as the gas flow rate. As for non-invasive ventilation, due to characteristics of its ventilation mode, a gas leakage may occur. Therefore, the gas flow rate obtained by the flow sensor 70 may be compensated to obtain an actual flow rate. Specifically, the processor 40 obtains a leakage amount during non-invasive ventilation, and then compensates the gas flow rate obtained by the flow sensor 70 according to the leakage amount, so as to obtain the actual gas flow rate.

This disclosure proposes a method for calculating a driving pressure without requiring manual operation by the user and interfering with ventilation. The measurement methods of inspiration pause or inspiratory hold are replaced by calculating an intrapulmonary pressure and using a calculated end-inspiratory intrapulmonary pressure as a plateau pressure; while the expiratory hold method is replaced by using a calculated end-expiratory intrapulmonary pressure as a total positive end-expiratory pressure PEEPtotal (PEEPi+PEEP); and then a driving pressure is obtained by subtracting the end-expiratory intrapulmonary pressure from an end-inspiratory intrapulmonary pressure. Since there is no need for inspiratory hold and expiratory hold, a real-time driving pressure can be calculated in real time, thereby realizing real-time monitoring of the driving pressure. The monitoring of the driving pressure can be real-time or not. This embodiment is described by taking real-time monitoring as an example. The method is shown in FIG. 4 and includes following steps.

In step 1, according to airway pressure data which are obtained by a pressure sensor in a respiratory cycle and gas flow rate data which are measured by a flow sensor in said respiratory cycle, calculate an intrapulmonary pressure of an inspiratory phase of a patient and an expiratory pressure of said patient, which pressures correspond to said respiratory cycle. In this embodiment, as an example, the intrapulmonary pressure of the inspiratory phase uses an end-inspiratory intrapulmonary pressure, and the expiratory pressure uses an end-expiratory intrapulmonary pressure. Specifically, taking real-time monitoring as an example, the processor 40 obtains the gas flow rate data of a patient during a ventilation process of a respiratory cycle in real time through the flow sensor 70, and obtains the airway pressure data of the patient during the ventilation process of the respiratory cycle in real time through the second pressure sensor 60. Then, the processor 40 calculates the intrapulmonary pressure data which correspond to the respiratory cycle, according to the gas flow rate data and the airway pressure data which correspond to the respiratory cycle; wherein the intrapulmonary pressure data at least includes an end-inspiratory intrapulmonary pressure and an end-expiratory intrapulmonary pressure.

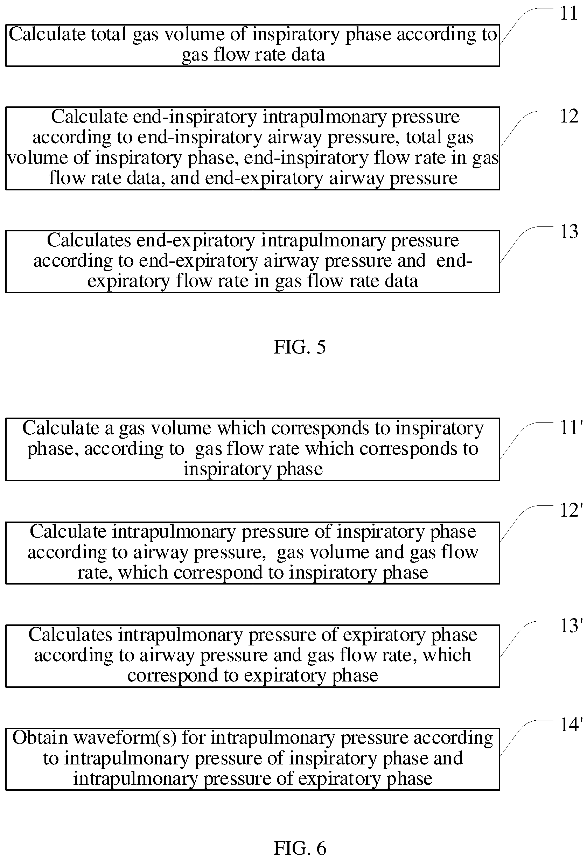

The processor 40 may calculate real-time intrapulmonary pressure data in a manner shown in FIG. 5, wherein the airway pressure data includes an end-inspiratory airway pressure $Paw(t_{EI})$ and an end-expiratory airway pressure $Paw(t_{EE})$. The method shown in FIG. 5 is described in detail below, which includes following steps.

In step 11, the processor 40 calculates a total gas volume $V(t_{EI})$ of the inspiratory phase according to the gas flow rate data. The total gas volume $V(t_{EI})$ of the inspiratory phase represents a total volume of gas which enters lungs of the patient during the inspiratory phase of the respiratory cycle, and can be obtained by integrating the gas flow rate data.

In step 12, the processor 40 calculates an end-inspiratory intrapulmonary pressure Plung ($t_{EI}$) according to the end-inspiratory airway pressure Paw ($t_{EI}$), the total gas volume V($t_{EI}$) of the inspiratory phase, an end-inspiratory flow rate Flow ($t_{EI}$) in the gas flow rate data, and the end-expiratory airway pressure Paw ($t_{EE}$).

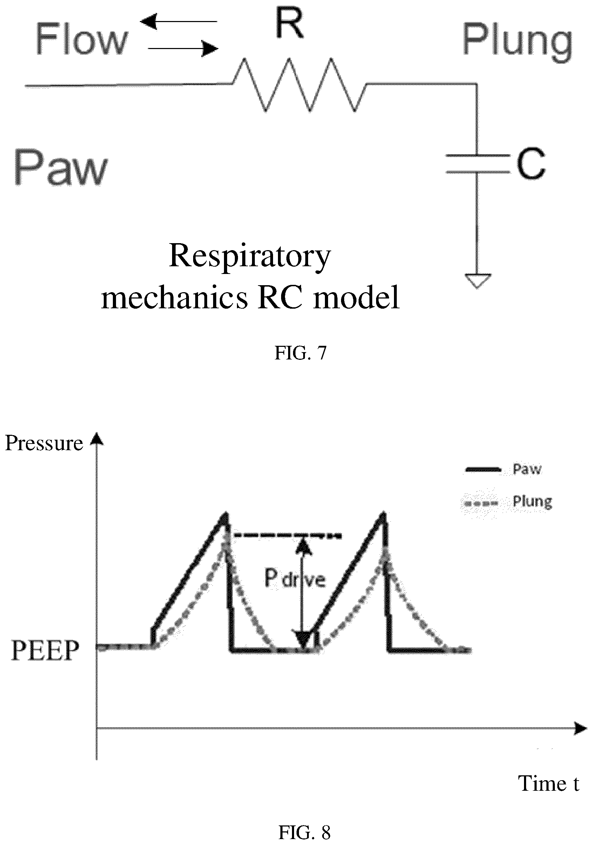

Specifically, as shown in FIG. 7, a respiratory mechanics RC model reveals relationships between various parameters. Wherein Flow is the gas flow rate, which is equivalent to a current; Paw is the airway pressure, which is equivalent to a voltage; an airway pressure resistance is equivalent to a resistance R; compliance of the respiratory system is equivalent to capacitance C; Plung is the intrapulmonary pressure. The respiratory mechanics equation can be constructed as follows:

$$Paw(t) = Flow(t) * R + \frac{\int Flow(t)dt}{C} = Flow(t) * R + \frac{V(t)}{C} \ ;$$

Inspiratory phase:

Expiratory phase: Plung(t)=Paw(t)-R*Flow(t).

Wherein, R is a resistance of the respiratory system, which is a known quantity or a preset quantity; t is a time or a time point, Paw(t) is a function of airway pressure and time t, that is, an airway pressure at any time t; Flow(t) is a function of gas flow rate and time t, that is, a gas flow rate at any time t; V(t) is a function of gas volume and time t, that is, a gas volume at any time t; wherein V(t) can be obtained by integrating the gas flow rate Flow(t). A gas volume at time t represents a total volume of gas that enters the body of the patient, from time when inhalation starts to the current time t. Plung(t) is a function of intrapulmonary pressure and time t, that is, an intrapulmonary pressure at any time t.

Intrapulmonary pressure, which means a pressure inside the lungs, is usually not directly measurable. In the prior art, the purposes of inspiratory hold and expiratory hold are to make the airway pressure equal to the intrapulmonary pressure after an airflow in the airway stabilizes, and to remove an interference of airway resistance, so as to use the airway pressure as the intrapulmonary pressure.

This disclosure does not perform inspiratory hold and expiratory hold, but uses following formula to calculate the intrapulmonary pressure:

Inspiratory phase:

$$\text{Plung(t)} = \frac{V(t)*[Paw(t)-PEEP]}{V(t)+\tau*Flow(t)}+\text{PEEP, formula (1);}$$

Expiratory phase:

$$\text{Plung(t)=Paw(t)-R*Flow(t) , formula (2).}$$

Wherein PEEP is the positive end-expiratory pressure, that is, the end-expiratory airway pressure Paw ($t_{EE}$), usually with a unit of cmH$_2$O; $\tau$ is a time constant with a value which is affected by resistance and compliance of the patient, and reflects how fast the patient exhales. Clinically, it is believed that the average expiratory time required for a patient to complete exhalation is 3 times of the time constant. There are many methods for calculating the time constant. One calculation method is to use a ratio of the tidal volume to the flow rate at a certain time point in the expiratory phase as the time constant. Another calculation method is to calculate the time constant by fitting a real-time volume-time curve. The obtained value may be different depending on the calculation methods. The time constant can be preset and this disclosure does not limit it.

The processor 40 substitutes the end-inspiratory airway pressure Paw ($t_{EI}$), the total gas volume V($t_{EI}$) of the inspiratory phase, the end-inspiratory flow rate Flow ($t_{EI}$) and the end-expiratory airway pressure Paw ($t_{EE}$) (i.e., PEEP) into the above formula (1) to calculate the end-inspiratory intrapulmonary pressure Plung ($t_{EI}$); wherein $t_{EI}$ is a time point of inspiratory end.

In step 13, the processor 40 calculates the end-expiratory intrapulmonary pressure Plung ($t_{EE}$) according to the end-expiratory airway pressure Paw ($t_{EE}$) and an end-expiratory flow rate Flow ($t_{EE}$) in the gas flow rate data. Specifically, the processor 40 substitutes the end-expiratory airway pressure Paw($t_{EE}$) and the end-expiratory flow rate Flow($t_{EE}$) into the above formula (2) to calculate the end-expiratory intrapulmonary pressure Plung($t_{EE}$); wherein $t_{EE}$ is a time point of expiratory end. Of course, in some embodiments, the positive end-expiratory pressure PEEP, that is, the end-expiratory airway pressure Paw($t_{EE}$) can also be used as the end-expiratory intrapulmonary pressure. Using PEEP as an approximate value of the end-expiratory intrapulmonary pressure to calculate the driving pressure, is slightly less accurate than formula (2).

The intrapulmonary pressure data may also include intrapulmonary pressures at different time points. Specifically, the processor 40 may calculate the real-time intrapulmonary pressure data in the manner shown in FIG. 6. Wherein the gas flow rate data includes, in a respiratory cycle, a gas flow rate which corresponds to the inspiratory phase (gas flow rates at different time points in the inspiratory phase), a gas flow rate, which corresponds to the expiratory phase (gas flow rates at different time points in the expiratory phase), that is, Flow(t) is known. The airway pressure data include an end-expiratory airway pressure PEEP, an airway pressure which corresponds to the inspiratory phase (airway pressures at different time points in the inspiratory phase), and an airway pressure which corresponds to the expiratory phase (airway pressures at different time points in the expiratory phase), that is, Paw(t) is known. The method shown in FIG. 6 is described in detail below, which includes following steps.

In step 11', the processor 40 calculates a gas volume $V(t_{IN})$ which corresponds to the inspiratory phase, according to a gas flow rate $Flow(t_{IN})$ which corresponds to the inspiratory phase. Wherein $t_{IN}$ represents different time points in the inspiration phase.

In step 12', the processor 40 calculates an intrapulmonary pressure Plung $(t_{IN})$ of the inspiratory phase, according to the airway pressure Paw $(t_{IN})$, the gas volume $V(t_{IN})$ and the gas flow rate Flow $(t_{IN})$, which three correspond to the inspiratory phase. Specifically, the end-expiratory airway pressure PEEP can also be added to calculate Plung $(t_{IN})$. For example, the processor 40 substitutes the end-expiratory airway pressure PEEP, and the airway pressure Paw $(t_{IN})$, the gas volume $V(t_{IN})$ and the gas flow rate Flow $(t_{IN})$, which are respectively at different time points in the inspiratory phase, into the above formula (1) to calculate the intrapulmonary pressure Plung $(t_{IN})$ of the inspiratory phase (intrapulmonary pressures at different time points in the inspiratory phase).

In step 13', the processor 40 calculates an intrapulmonary pressure Plung $(t_{EN})$ of the expiratory phase, according to the airway pressure Paw $(t_{EN})$ and the gas flow rate Flow $(t_{EN})$, which correspond to the expiratory phase. Specifically, the processor 40 substitutes the airway pressure Paw $(t_{EN})$ which corresponds to the expiratory phase and the gas flow rate Flow $(t_{EN})$ which corresponds to the expiratory phase into the above formula (2) to calculate the intrapulmonary pressure Plung $(t_{EN})$ of the expiratory phase (intrapulmonary pressures at different time points in the expiratory phase). Wherein $t_{EN}$ represents different time points in the expiratory phase.

In step 14', the processor 40 obtains intrapulmonary pressures Plung(t) of the patient at different time points in the entire respiratory cycle, according to the intrapulmonary pressure Plung $(t_{IN})$ of the inspiratory phase and the intrapulmonary pressure Plung $(t_{EN})$ of the expiratory phase, so as to obtain waveform(s) for intrapulmonary pressure(s), and display the same through the display, as shown in the dotted waveform(s) in FIG. 8. In this way, the user can see a trend of change for the intrapulmonary pressure over time, making it easier to understand a respiratory condition of the patient. Of course, the processor 40 may also generate airway pressure waveform(s) according to the airway pressure data, and display the same through a display, as shown by solid line waveform(s) in FIG. 8. The waveform(s) for intrapulmonary pressure(s) and the airway pressure waveform(s) are in a same graph, sharing the same coordinate axis, making it easier for users to view.

In step 2, the processor 40 subtracts the end-expiratory intrapulmonary pressure Plung $(t_{EE})$ from the end-inspiratory intrapulmonary pressure Plung $(t_{EI})$ to obtain a real-time driving pressure Pdrive; that is, Pdrive=Plung $(t_{EI})$-Plung $(t_{EE})$. The driving pressure Pdrive is a driving force that drives the entire respiratory system of the patient to expand, that is, a driving pressure which acts on the lungs and chest wall.

This disclosure creatively adopts two different formulas to derive and calculate the end-inspiratory intrapulmonary pressure Plung $(t_{EI})$ and the end-expiratory intrapulmonary pressure Plung $(t_{EE})$, and uses a difference between the end-inspiratory intrapulmonary pressure Plung $(t_{EI})$ and the end-expiratory intrapulmonary pressure Plung $(t_{EE})$ as the driving pressure, which does not require inspiratory hold and expiratory hold.

The real-time driving pressure Pdrive obtained by the processor 40 can be further utilized or outputted. For example, the processor 40 analyses the driving pressure based on a first preset condition. When the driving pressure does not satisfy the first preset condition, the processor 40 outputs a corresponding indication signal, such as outputting indication information to the display apparatus 30. The first preset condition may be a preset pressure range. If the driving pressure does not fall into the pressure range, it means that the driving pressure is too high or too low. Then the processor 40 outputs a corresponding indication signal to remind the doctor. When the processor 40 fails to calculate the driving pressure, it may also output a corresponding indication signal. For example, if the processor 40 cannot calculate the driving pressure, it outputs a corresponding indication signal. If the processor 40 reports an error during the calculation of the driving pressure or the obtained driving pressure exceeds an error threshold (such as the error threshold is a value that the driving pressure cannot reach), it outputs a corresponding indication signal. The indication signal may be alarming information, which is given by a display apparatus, a speaker, or an indicator light.

The processor 40 may also adjust the ventilation of the patient according to the driving pressure, for example, by adjusting the driving pressure through a flow or pressure control. For example, the driving pressure can be outputted to an external device (such as a printer, a monitor, etc.), or to a display apparatus 30 which displays, on its display interface, the real-time driving pressure Pdrive, such as displaying a real-time value, displaying the driving pressure in a form of a graph or chart that changes over time, etc. The processor 40 may also obtain a trend of change over time for the driving pressure, according to the driving pressure Pdrive at different time points, for example, to obtain a waveform diagram of the driving pressure Pdrive. The processor 40 further transmits the trend of change over time for the driving pressure to the display apparatus 30. The display apparatus 30 receives the trend of change over time for the driving pressure transmitted by the processor 40, and displays the same on its display interface, for example, displaying a waveform diagram of the driving pressure Pdrive, so that medical staff can understand the changes in the driving pressure of the patient. Since the driving pressure can be monitored in real time, the safety of patient ventilation is improved.

Furthermore, the processor 40 can also obtain a tidal volume (TV) of the patient in real time. For example, the processor 40 obtains the tidal volume of the patient in real time through the flow sensor, and obtains the corresponding compliance according to the tidal volume and the driving pressure, such as calculating the ratio of the tidal volume to the driving pressure to obtain the real-time compliance, that is, compliance = tidal volume/driving pressure. The processor 40 may also monitor the respiratory state of the patient based on the compliance. Compliance refers to a difficulty level for an elastic body to deform under an action of an external force. The processor 40 displays the compliance through the display apparatus 30, so that medical staff can understand the physiological condition of the lungs of the patient and thus better ventilate the patient.

Airway pressure is a pressure which acts on the respiratory system (an airway resistance, lungs, and chest wall, etc.). Intrapulmonary pressure is a pressure which acts directly on the lungs and chest wall after removing the airway resistance. Transpulmonary pressure is a pressure which acts directly on the lungs, which is a result of removing a part of the intrapulmonary pressure, which part acts on the chest wall. The transpulmonary pressure is obtained by subtracting an intrathoracic pressure from the intrapulmonary pressure. The intrathoracic pressure is usually approximated by an esophageal pressure, that is, transpulmonary pressure = intrapulmonary pressure - esophageal pressure. After the processor 40 calculates real-time intrapulmonary pressure data according to the gas flow rate data and the airway pressure data which correspond to the respiratory cycle, it can also calculate difference(s) between the intrapulmonary pressure data and the esophageal pressure data, so as to obtain the real-time transpulmonary pressure data. The transpulmonary pressure data includes an end-inspiratory transpulmonary pressure and an end-expiratory transpulmonary pressure. A real-time transpulmonary driving pressure is obtained by subtracting the end-expiratory transpulmonary pressure from the end-inspiratory transpulmonary pressure. Specifically, the intrapulmonary pressure data at least includes an end-inspiratory intrapulmonary pressure and an end-expiratory intrapulmonary pressure, the esophageal pressure data at least includes an end-inspiratory esophageal pressure and an end-expiratory esophageal pressure. The processor 40 subtracts the end-inspiratory esophageal pressure from the end-inspiratory intrapulmonary pressure to obtain a real-time end-inspiratory transpulmonary pressure; and subtracts the end-expiratory esophageal pressure from the end-expiratory intrapulmonary pressure to obtain a real-time end-expiratory transpulmonary pressure. Of course, the intrapulmonary pressure data may also include intrapulmonary pressures at different time points, such as waveform(s) for intrapulmonary pressure(s); the esophageal pressure data may also include esophageal pressures at different time points, such as esophageal pressure waveform(s); the transpulmonary pressure data may also include transpulmonary pressures at different time points, such as transpulmonary pressure waveform(s). The processor 40 subtracts esophageal pressure waveform(s) from waveform(s) for intrapulmonary pressure(s) to obtain transpulmonary pressure waveform(s). The end-inspiratory transpulmonary pressure and the end-expiratory transpulmonary pressure can be obtained based on the transpulmonary pressure waveform(s), and a difference value between the end-inspiratory transpulmonary pressure and the end-expiratory transpulmonary pressure is the transpulmonary driving pressure.

In some embodiments, the airway pressure data may be used to approximately replace esophageal pressure data to calculate transpulmonary pressure data, that is, the difference value between the intrapulmonary pressure data and the airway pressure data is calculated to obtain the real-time transpulmonary pressure data. For example, the processor 40 subtracts the end-inspiratory airway pressure from the end-inspiratory intrapulmonary pressure to obtain the real-time end-inspiratory transpulmonary pressure; subtracts the end-expiratory airway pressure from the end-expiratory intrapulmonary pressure to obtain the real-time end-expiratory transpulmonary pressure. For another example, the processor 40 subtracts the intrapulmonary pressure waveform(s) from the airway pressure waveform(s) to obtain the transpulmonary pressure waveform(s), etc. The specific process of using the airway pressure data to calculate the transpulmonary pressure data is the same as the above-mentioned process of using the esophageal pressure data to calculate the transpulmonary pressure data, and is not repeated here.

The processor 40 can also display, on the display interface of the display apparatus 30, a real-time transpulmonary driving pressure to provide medical staff with a reference for the force acting on the lungs; can also display, on the display interface of the display apparatus 30, the trend of change over time for the transpulmonary driving pressure, according to the transpulmonary driving pressures obtained at different time points, such as displaying a waveform graph of the transpulmonary driving pressure.

The processor 40 can obtain corresponding lung compliance according to the tidal volume and the transpulmonary driving pressure, such as to calculate a ratio of the tidal volume to the transpulmonary driving pressure, so as to obtain the real-time lung compliance, that is, lung compliance = tidal volume / transpulmonary driving pressure. The processor 40 may also monitor the respiratory state of the patient according to the lung compliance. The processor 40 can also display the lung compliance on the display interface of the display apparatus 30, so that medical staff can understand the elasticity of the lungs of the patient.

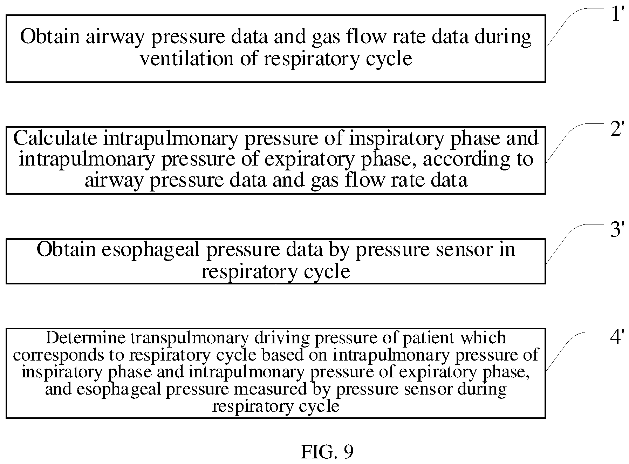

In some embodiments, the respiration monitoring apparatus may monitor the transpulmonary driving pressure. The respiratory monitoring process is shown in FIG. 9 and includes following steps.

In step 1', the processor 40 obtains airway pressure data measured by the pressure sensor and gas flow rate data measured by the flow sensor in a respiratory cycle. For example, the airway pressure data and the gas flow rate data of the patient during a ventilation process are obtained in real time The specific process has been described in detail in the above embodiments and is not repeated here.

In step 2', according to airway pressure data which are measured in a respiratory cycle and gas flow rate data which are measured in said respiratory cycle, the processor 40 calculates an intrapulmonary pressure of an inspiratory phase of a patient and an intrapulmonary pressure of an expiratory phase of said patient, which pressures correspond to the respiratory cycle. For example, real-time intrapulmonary pressure data, which includes the intrapulmonary pressure of the inspiratory phase and the intrapulmonary pressure of the expiratory phase, can be obtained by the method shown in FIG. 5 or FIG. 6. The specific process has been described in detail in the above embodiments and is not repeated here.

Step 3', the processor 40 obtains esophageal pressure data of the patient in real time. For example, the processor 40 detects an esophageal pressure of the patient in real time through the first pressure sensor 50 to obtain the esophageal pressure data.

Step 4', the processor 40 determines a transpulmonary driving pressure of the patient which corresponds to said respiratory cycle based on the intrapulmonary pressure of the inspiratory phase and the intrapulmonary pressure of the expiratory phase, and an esophageal pressure which is measured by the pressure sensor in said respiratory cycle. There are several ways to determine the transpulmonary driving pressure, which are described below.

One way is that in step 2', the processor 40 calculates, according to the airway pressure data which is obtained by the pressure sensor in said respiratory cycle, and the gas flow rate data which is measured by the flow sensor in said respiratory cycle, the end-inspiratory intrapulmonary pressure of the patient and the end-expiratory intrapulmonary pressure of the patient, which pressures correspond to the respiratory cycle. The specific process has been described in detail in the above embodiment and is not repeated here.

Another way is that in step 2', the processor 40 calculates, according to the airway pressure data which is obtained by the pressure sensor in said respiratory cycle, and the gas flow rate data which is measured by the flow sensor in said respiratory cycle, an intrapulmonary pressure curve for the inspiratory phase of the patient and an intrapulmonary pressure curve for the expiratory phase of the patient, which curves correspond to said respiratory cycle. The specific process has been described in detail in the above embodiment and is not repeated here.

In the above two methods, the processor 40 can specifically calculate the transpulmonary driving pressure in following ways.

For one respiratory cycle, the processor 40 calculates intrapulmonary pressure data at an inspiratory end of said respiratory cycle, and intrapulmonary pressure data at an expiratory end of said respiratory cycle, according to the gas flow rate data which correspond to said respiratory cycle and the airway pressure data which correspond to said respiratory cycle; obtain transpulmonary pressure data which respectively correspond to the inspiratory end of said respiratory cycle and the expiratory end of said respiratory cycle, according to difference(s) between the intrapulmonary pressure data at the inspiratory end and esophageal pressure data which are measured by the pressure senor at corresponding time point(s), and difference(s) between the intrapulmonary pressure data at the expiratory end and esophageal pressure data which are measured by the pressure senor at corresponding time point(s).

The intrapulmonary pressure data may include waveform(s) for intrapulmonary pressure (s), the esophageal pressure data may include waveform(s) for esophageal pressure(s), and the transpulmonary pressure data may include waveform(s) for transpulmonary pressure(s). The end-inspiratory transpulmonary pressure and the end-expiratory transpulmonary pressure can be obtained based on the waveform(s) for transpulmonary pressure(s).

The intrapulmonary pressure data may also include an end-inspiratory intrapulmonary pressure and an end-expiratory intrapulmonary pressure, and the esophageal pressure data may include an end-inspiratory esophageal pressure and an end-expiratory esophageal pressure. The processor 40 calculates differences between the intrapulmonary pressure data and the esophageal pressure data to obtain transpulmonary pressure data which correspond to the respiratory cycle. The method can be as follows subtracting the end-inspiratory esophageal pressure from the end-inspiratory intrapulmonary pressure to obtain the corresponding end-inspiratory transpulmonary pressure; and subtracting the end-expiratory esophageal pressure from the end-expiratory intrapulmonary pressure to obtain the corresponding end-expiratory transpulmonary pressure.

Furthermore, the processor 40 obtains a transpulmonary driving pressure which corresponds to the respiratory cycle based on the end-inspiratory transpulmonary pressure and the end-expiratory transpulmonary pressure, for example, the transpulmonary driving pressure which corresponds to the respiratory cycle is obtained by subtracting the end-expiratory transpulmonary pressure from the end-inspiratory transpulmonary pressure. The corresponding transpulmonary driving pressure can be calculated for each respiratory cycle, thereby performing real-time detection of the transpulmonary driving pressure. The specific process has been described in detail in the above embodiments and is not repeated here.

The real-time transpulmonary driving pressure obtained by the processor 40 can be further utilized or outputted. For example, the processor 40 analyses the transpulmonary driving pressure based on a second preset condition. When the transpulmonary driving pressure does not satisfy the second preset condition, the processor 40 outputs a corresponding indication signal, such as outputting indication information to the display apparatus 30. The second preset condition may be a preset pressure range. If the transpulmonary driving pressure does not fall into this pressure range, it means that the transpulmonary driving pressure is too high or too low, and the processor 40 outputs a corresponding indication signal to remind the doctor. When the processor 40 fails to calculate the transpulmonary driving pressure, it may also output a corresponding indication signal. For example, if the processor 40 cannot calculate the transpulmonary driving pressure, it outputs a corresponding indication signal. If the processor 40 reports an error during the calculation of the transpulmonary driving pressure or the obtained transpulmonary driving pressure exceeds an error threshold (such as the error threshold is a value that the transpulmonary driving pressure cannot reach), it outputs a corresponding indication signal. The indication signal may be alarming information, which is given by a display apparatus, a speaker, or an indicator light.

The processor 40 may also adjust the ventilation of the patient according to the transpulmonary driving pressure, for example, by adjusting the transpulmonary driving pressure through a flow or pressure control. For another example, the processor 40 may output the transpulmonary driving pressure to an external device (such as a printer, a monitor, etc.), or to a display apparatus 30, and the display interface of the display apparatus 30 displays the real-time transpulmonary driving pressure, such as displaying a real-time numerical value, displaying the transpulmonary driving pressure in a form of a graph or chart that changes over time, etc. The processor 40 can also obtain a trend of change over time for the transpulmonary driving pressure based on the transpulmonary driving pressure at different time points, such as obtaining a waveform graph of the transpulmonary driving pressure; the processor 40 transmits the trend of change over time for the transpulmonary driving pressure to the display apparatus 30 for displaying, such as for displaying a waveform graph of the transpulmonary driving pressure, so that medical staff can understand the changes in a transpulmonary driving pressure of the patient.

In summary, in the respiration monitoring method and respiration monitoring apparatus provided by this disclosure, the driving pressure can be calculated and monitored in real time by using the intrapulmonary pressure, without requiring inspiratory hold or manual operation by the user; the plateau pressure and PEEPtotal can be calculated and monitored in real time in the process of calculating the driving pressure, without requiring inspiratory hold or manual operation by the user; the transpulmonary driving pressure can be calculated and monitored in real time, without requiring inspiratory hold or manual operation by the user; the compliance and lung compliance can be calculated and monitored in real time based on the driving pressure, without requiring inspiratory hold or manual operation by the user.

Those skilled in the art can understand that all or part of the functions of various methods in the above embodiments can be implemented by hardware or by computer programs. When all or part of the functions in the above embodiments are implemented by a computer program, the program can be stored in a computer-readable storage medium. The storage medium can include a read-only memory, a random-access memory, a magnetic disk, an optical disk, a hard disk, etc., and the above functions can be achieved through execution of this program through a computer. For example, the program is stored in a device memory, and when the program in the memory is executed by the processor, all or part of the above functions can be realized. In addition, when all or part of the functions in the above embodiments are implemented by a computer program, the program can also be stored in a storage medium, such as a server, another computer, a magnetic disk, an optical disk, a flash disk or a mobile hard disk, and can be downloaded or copied to save it into the memory of the local device, or to perform a version update on the system of the local device. When the program in the memory is executed by the processor, all, or part of the functions in the above embodiments can be realized.

The description has been made with reference to various exemplary embodiments herein. However, those skilled in the art recognize that changes and modifications can be made to the exemplary embodiments without departing from the scope herein. For example, various operation steps and components for performing operation steps may be implemented in different ways according to a specific application or considering any number of cost functions associated with the operation of the system (for example, one or more steps may be deleted, modified, or incorporated into other steps).

In addition, as understood by those skilled in the art, the principles herein may be reflected in a computer program product on a computer-readable storage medium that is pre-installed with computer-readable program codes. Any tangible, non-transitory computer-readable storage medium can be used, including magnetic storage devices (hard disks, floppy disks, etc.), optical storage devices (CD-ROM, DVD, Blu Ray disks, etc.), flash memory, and/or the like. These computer program instructions can be loaded onto a general-purpose computer, a dedicated computer, or other programmable data processing device to form a machine, so that these instructions, which are executed on a computer or other programmable data processing apparatus, can generate an apparatus that implements a specified function. These computer program instructions can also be stored in a computer-readable memory that can instruct a computer or other programmable data processing device to operate in a specific manner, so that the instructions stored in the computer-readable memory can form a manufactured product, including an implementation apparatus that implements a specified function. The computer program instructions can also be loaded onto a computer or other programmable data processing device, so that a series of operating steps are performed on the computer or other programmable device to produce a computer-implemented process, so that the instructions executed on a computer or other programmable data processing device can provide steps for implementing specified functions.

Although the principles herein have been shown in various embodiments, many modifications of structures, arrangements, proportions, elements, materials, and components particularly suitable for the specific environmental and operational requirements may be used without departing from the principles and scope of the present disclosure. The above modifications and other changes or amendments will be included in the scope of this disclosure.

The foregoing specific description has been described with reference to various embodiments. However, those skilled in the art recognize that various modifications and changes can be made without departing from the scope of the present disclosure. Therefore, consideration of the present disclosure is in an illustrative rather than a restrictive sense, and all such modifications are included within the scope thereof. Also, the advantages of various embodiments, other advantages, and the solutions to problems have been described above. However, the benefits, advantages, solutions to problems, and any elements that can produce these, or solutions that make them more explicit, should not be interpreted as critical, necessary, or essential. The term "including", and any other variants thereof used herein are non-exclusive, so that the process, method, document, or device that includes a list of elements includes not only these elements, but also other elements that are not explicitly listed or do not belong to the process, method, system, document, or device. Furthermore, the term "coupling" and any other variations thereof used herein refer to physical connection, electrical connection, magnetic connection, optical connection, communicational connection, functional connection, and/or any other connection.

It should be noted that a person of ordinary skill in the art could also make several variations and improvements without departing from the concept of this disclosure, and these variations and improvements would all fall within the scope of protection of this disclosure. Therefore, the protection scope of this disclosure should be in accordance with the appended claims.

**Claims**

1. A respiration monitoring apparatus, **characterized in that**, comprising:

   a pressure sensor, which is configured to obtain an airway pressure of a patient, or obtain an airway pressure and an esophageal pressure of a patient, so as to obtain corresponding airway pressure data, or obtain corresponding airway pressure data and esophageal pressure data;
   a flow sensor, which is configured to obtain gas flow rate data of a patient during a ventilation process;
   a processor, which is configured to:

   according to airway pressure data which are measured by the pressure sensor in a respiratory cycle and gas flow rate data which are measured by the flow sensor in said respiratory cycle, calculate an intrapulmonary pressure of an inspiratory phase of a patient and an expiratory pressure of said patient, which pressures correspond to said respiratory cycle; and determine a driving pressure of said patient, which pressure corresponds to said respiratory cycle, according to the intrapulmonary pressure of the inspiratory phase and the expiratory pressure; or
   according to airway pressure data which are measured by the pressure sensor in a respiratory cycle and gas flow rate data which are measured by the flow sensor in said respiratory cycle, calculate an intrapulmonary pressure of an inspiratory phase of a patient and an intrapulmonary pressure of an expiratory phase of said patient, which pressures correspond to said respiratory cycle; and determine a transpulmonary driving pressure of said patient, which pressure corresponds to said respiratory cycle, according to the intrapulmonary pressure of the inspiratory phase and the intrapulmonary pressure of the expiratory phase, as well as esophageal pressure data which are measured by the pressure sensor in said respiratory cycle.

2. The respiration monitoring apparatus according to claim 1, **characterized in that**, in order to determine the transpulmonary driving pressure of said patient, the processor is further configured to:

   according to the airway pressure data which are measured by the pressure sensor in said respiratory cycle and the gas flow rate data which are measured by the flow sensor in said respiratory cycle, calculate an end-inspiratory intrapulmonary pressure of said patient and an end-expiratory intrapulmonary pressure of said patient, which pressures correspond to said respiratory cycle; and determine the transpulmonary driving pressure of said patient, which pressure corresponds to said respiratory cycle, according to the end-inspiratory intrapulmonary pressure and the end-expiratory pulmonary pressure, as well as the esophageal pressure data which are measured by the pressure sensor in said respiratory cycle; or

according to the airway pressure data which are measured by the pressure sensor in said respiratory cycle and the gas flow rate data which are measured by the flow sensor in said respiratory cycle, calculate an intrapulmonary pressure curve for the inspiratory phase of said patient and an intrapulmonary pressure curve for the expiratory phase of said patient, which curves correspond to said respiratory cycle; and determine a transpulmonary pressure curve which corresponds to said respiratory cycle, according to the intrapulmonary pressure curve for the inspiratory phase and the intrapulmonary pressure curve for the expiratory phase, as well as an esophageal pressure curve which is measured by the pressure sensor in said respiratory cycle; determine an end-inspiratory transpulmonary pressure and an end-expiratory transpulmonary pressure according to the transpulmonary pressure curve, so as to determine the transpulmonary driving pressure of said patient, which pressure corresponds to said respiratory cycle.

3. The respiration monitoring apparatus according to claim 1 or claim 2, **characterized in that**, further comprising a display apparatus, wherein the processor is further configured to transmit the driving pressure or the transpulmonary driving pressure to the display apparatus for displaying.

4. The respiration monitoring apparatus according to claim 3, **characterized in that**, the driving pressure or the transpulmonary driving pressure is displayed on the display apparatus in a graph which changes with time.

5. The respiration monitoring apparatus according to claim 1 or claim 2, **characterized in that**, the intrapulmonary pressure of the inspiratory phase comprises an end-inspiratory intrapulmonary pressure, the intrapulmonary pressure of the expiratory phase comprises an end-expiratory intrapulmonary pressure;
in order to calculate the end-inspiratory intrapulmonary pressure and the end-expiratory intrapulmonary pressure, the processor is further configured to:

obtain, in real time, the gas flow rate data which are measured by the flow sensor during a ventilation process of said respiratory cycle of said patient, and the airway pressure data which are measured by the pressure sensor during said ventilation process of said respiratory cycle of said patient;
calculate intrapulmonary pressure data, which correspond to said respiratory cycle, according to the gas flow rate data and the airway pressure data which correspond to said respiratory cycle; wherein the intrapulmonary pressure data comprise the end-inspiratory intrapulmonary pressure and the end-expiratory intrapulmonary pressure.

6. The respiration monitoring apparatus according to claim 1 or claim 2, **characterized in that**, in order to determine the transpulmonary driving pressure of said patient, the processor is further configured to:

for said respiratory cycle, calculate intrapulmonary pressure data at an inspiratory end of said respiratory cycle, and intrapulmonary pressure data at an expiratory end of said respiratory cycle, according to the gas flow rate data and the airway pressure data which correspond to said respiratory cycle;
obtain transpulmonary pressure data which respectively correspond to the inspiratory end of said respiratory cycle and the expiratory end of said respiratory cycle, according to difference(s) between the intrapulmonary pressure data at the inspiratory end and esophageal pressure data which are measured by the pressure senor at corresponding time point(s), and difference(s) between the intrapulmonary pressure data at the expiratory end and esophageal pressure data which are measured by the pressure senor at corresponding time point(s); wherein the transpulmonary pressure data comprise an end-inspiratory transpulmonary pressure and an end-expiratory transpulmonary pressure;
obtain the transpulmonary driving pressure which corresponds to said respiratory cycle, according to the end-inspiratory transpulmonary pressure and the end-expiratory transpulmonary pressure.

7. The respiration monitoring apparatus according to claim 6, **characterized in that**, the intrapulmonary pressure data comprise waveform(s) for intrapulmonary pressure(s), the esophageal pressure data comprise waveform(s) for esophageal pressure(s), and the transpulmonary pressure data comprise waveform(s) for transpulmonary pressure(s); or

the intrapulmonary pressure data comprise an end-inspiratory intrapulmonary pressure and an end-expiratory intrapulmonary pressure, and the esophageal pressure data comprise an end-inspiratory esophageal pressure and an end-expiratory esophageal pressure;

in order to calculate differences between the intrapulmonary pressure data and the esophageal pressure data, so as to obtain the intrapulmonary pressure data which correspond to said respiratory cycle, the processor is further configured to:

subtract the end-inspiratory esophageal pressure from the end-inspiratory intrapulmonary pressure to obtain a corresponding end-inspiratory transpulmonary pressure; and
subtract the end-expiratory esophageal pressure from the end-expiratory intrapulmonary pressure to obtain a corresponding end-expiratory transpulmonary pressure.

8. The respiration monitoring apparatus according to claim 5, claim 6 or claim 7, **characterized in that**:

the gas flow rate data comprise a gas flow rate which corresponds to the inspiratory phase of said respiratory cycle, and a gas flow rate which corresponds to the expiratory phase of said respiratory cycle; the airway pressure data comprise an airway pressure which corresponds to the inspiratory phase of said respiratory cycle, and an airway pressure which corresponds to the expiratory phase of said respiratory cycle;

in order to calculate intrapulmonary pressure data, which correspond to said respiratory cycle, according to the gas flow rate data and the airway pressure data which correspond to said respiratory cycle, the processor is further configured to:

calculate a gas volume which corresponds to the inspiratory phase, according to the gas flow rate which corresponds to the inspiratory phase;
calculate the intrapulmonary pressure of the inspiratory phase, according to the airway pressure which corresponds to the inspiratory phase, the gas volume which corresponds to the inspiratory phase, and the gas flow rate which corresponds to the inspiratory phase;
calculate the intrapulmonary pressure of the expiratory phase, according to the airway pressure which corresponds to the expiratory phase, and the gas flow rate which corresponds to the expiratory phase; and
obtain waveform(s) for the intrapulmonary pressure(s) of said patient in said respiratory cycle, according to the intrapulmonary pressure of the inspiratory phase and the intrapulmonary pressure of the expiratory phase;
or
the airway pressure data comprise an end-inspiratory airway pressure and an end-expiratory airway pressure;
in order to calculate intrapulmonary pressure data, which correspond to said respiratory cycle, according to the gas flow rate data and the airway pressure data which correspond to said respiratory cycle, the processor is further configured to:

calculate a total gas volume which corresponds to the inspiratory phase, according to the gas flow rate data;
calculate the end-inspiratory intrapulmonary pressure according to the end-inspiratory airway pressure, the total gas volume which corresponds to the inspiratory phase, an end-inspiratory flow rate in the gas flow rate data, and the end-expiratory airway pressure;
calculate the end-expiratory intrapulmonary pressure according to the end-expiratory airway pressure and an end-expiratory flow rate in the gas flow rate data, or approximate the end-expiratory intrapulmonary pressure with the end-expiratory airway pressure.

9. The respiration monitoring apparatus according to claim 1 or claim 2, **characterized in that**, the processor is further configured to:

obtain, through the flow sensor in real time, a tidal volume of said patient;
obtain corresponding compliance according to the tidal volume and the driving pressure, or obtain corresponding lung compliance according to the tidal volume and the transpulmonary driving pressure; and
monitor a respiratory state of said patient according to the compliance or the lung compliance.

10. The respiration monitoring apparatus according to claim 1 or claim 2, **characterized in that**, the processor is further configured to:

EP 4 454 688 A1

analyse the driving pressure or the transpulmonary driving pressure according to a preset condition; and transmit an indication signal, when the driving pressure or the transpulmonary driving pressure does not satisfy the preset condition.

11. A respiration monitoring method, **characterized in that**, comprising:

during a ventilation process in real time, obtaining airway pressure data and gas flow rate data of a patient, or obtaining airway pressure data, esophageal pressure data and gas flow rate data of a patient;

for a respiratory cycle, calculating corresponding intrapulmonary pressure data according to gas flow rate data and airway pressure data in said respiratory cycle, wherein the intrapulmonary pressure data comprise an end-inspiratory intrapulmonary pressure and an end-expiratory intrapulmonary pressure; wherein the end-inspiratory intrapulmonary pressure and the end-expiratory intrapulmonary pressure respectively characterize a pressure which acts on an expected position of a respiratory system of said patient at an inspiratory end and an expiratory end respectively during the ventilation process; and obtaining a driving pressure which corresponds to said respiratory cycle, according to the end-inspiratory intrapulmonary pressure and the end-expiratory intrapulmonary pressure; or

according to airway pressure data which are measured by a pressure sensor in a respiratory cycle and gas flow rate data which are measured by a flow sensor in said respiratory cycle, calculating an intrapulmonary pressure of an inspiratory phase of a patient and an intrapulmonary pressure of an expiratory phase of said patient, which pressures correspond to said respiratory cycle; and determining a transpulmonary driving pressure of said patient, which pressure corresponds to said respiratory cycle, according to the intrapulmonary pressure of the inspiratory phase and the intrapulmonary pressure of the expiratory phase, as well as esophageal pressure data which are measured by the pressure sensor in said respiratory cycle.

12. The respiration monitoring method according to claim 11, **characterized in that**, determining the transpulmonary driving pressure of said patient, comprises:

according to the airway pressure data which are measured by the pressure sensor in said respiratory cycle and the gas flow rate data which are measured by the flow sensor in said respiratory cycle, calculating an end-inspiratory intrapulmonary pressure of said patient and an end-expiratory intrapulmonary pressure of said patient, which pressures correspond to said respiratory cycle; and determining the transpulmonary driving pressure of said patient, which pressure corresponds to said respiratory cycle, according to the end-inspiratory intrapulmonary pressure and the end-expiratory pulmonary pressure, as well as the esophageal pressure data which are measured by the pressure sensor in said respiratory cycle; or

for said respiratory cycle, calculating an intrapulmonary pressure curve according to the airway pressure data and the gas flow rate data which correspond to said respiratory cycle; wherein the intrapulmonary pressure curve comprises an intrapulmonary pressure curve for the inspiratory phase and an intrapulmonary pressure curve for the expiratory phase; obtaining a transpulmonary pressure curve which corresponds to said respiratory cycle, according to the intrapulmonary pressure curve for the inspiratory phase and the intrapulmonary pressure curve for the expiratory phase, as well as a corresponding esophageal pressure curve which corresponds to said respiratory cycle; determining an end-inspiratory transpulmonary pressure and an end-expiratory transpulmonary pressure according to the transpulmonary pressure curve; and obtaining the transpulmonary driving pressure of said patient, which pressure corresponds to said respiratory cycle, according to the end-inspiratory transpulmonary pressure and the end-expiratory transpulmonary pressure.

13. The respiration monitoring method according to claim 11, **characterized in that**:

the intrapulmonary pressure data comprise waveform(s) for intrapulmonary pressure(s), the esophageal pressure data comprise waveform(s) for esophageal pressure(s), and the transpulmonary pressure data comprise waveform(s) for transpulmonary pressure(s); or

the intrapulmonary pressure data comprise an end-inspiratory intrapulmonary pressure and an end-expiratory intrapulmonary pressure, and the esophageal pressure data comprise an end-inspiratory esophageal pressure and an end-expiratory esophageal pressure;

calculating differences between the intrapulmonary pressure data and the esophageal pressure data, so as to obtain real-time intrapulmonary pressure data, comprising:

subtracting the end-inspiratory esophageal pressure from the end-inspiratory intrapulmonary pressure to obtain a corresponding end-inspiratory transpulmonary pressure; and

subtracting the end-expiratory esophageal pressure from the end-expiratory intrapulmonary pressure to obtain a corresponding end-expiratory transpulmonary pressure.

14. The respiration monitoring method according to claim 11, **characterized in that**:

the gas flow rate data comprise a gas flow rate which corresponds to the inspiratory phase, and a gas flow rate which corresponds to the expiratory phase; the airway pressure data comprise an end-expiratory airway pressure, an airway pressure which corresponds to the inspiratory phase, and an airway pressure which corresponds to the expiratory phase;
calculating intrapulmonary pressure data, according to the gas flow rate data and the airway pressure data which correspond to said respiratory cycle, comprises:

calculating a gas volume which corresponds to the inspiratory phase, according to the gas flow rate which corresponds to the inspiratory phase;
calculating intrapulmonary pressures at different time points of the inspiratory phase, according to the end-expiratory airway pressure, and airway pressures, gas volumes and gas flow rates, at different time points of the inspiratory phase;
calculating intrapulmonary pressures at different time points of the expiratory phase, according to airway pressures and gas flow rates, at different time points of the expiratory phase;
obtaining waveform(s) for intrapulmonary pressure(s), according to the intrapulmonary pressures at different time points of the inspiratory phase and the intrapulmonary pressures at different time points of the expiratory phase;
or
the airway pressure data comprise an end-inspiratory airway pressure and an end-expiratory airway pressure;
calculating intrapulmonary pressure data, according to the gas flow rate data and the airway pressure data which correspond to said respiratory cycle, comprises:

calculating a total gas volume which corresponds to the inspiratory phase according to the gas flow rate data;
calculating the end-inspiratory intrapulmonary pressure according to the end-inspiratory airway pressure, the total gas volume which corresponds to the inspiratory phase, an end-inspiratory flow rate in the gas flow rate data, and the end-expiratory airway pressure;
calculating the end-expiratory intrapulmonary pressure according to the end-expiratory airway pressure and an end-expiratory flow rate in the gas flow rate data, or using the end-expiratory airway pressure as the end-expiratory intrapulmonary pressure.

15. The respiration monitoring method according to claim 11, **characterized in that**, further comprising:

obtaining, in real time, a tidal volume of said patient;
obtaining corresponding compliance according to the tidal volume and the driving pressure, or obtaining corresponding lung compliance according to the tidal volume and the transpulmonary driving pressure; and
monitoring a respiratory state of said patient according to the compliance or the lung compliance.

16. The respiration monitoring method according to claim 11, **characterized in that**, further comprising at least one of:

displaying, on a display interface of a display apparatus, the driving pressure;
displaying, on a display interface of a display apparatus, a trend of change over time for the driving pressure, according to driving pressures at different time points;
displaying, on a display interface of a display apparatus, a real-time transpulmonary driving pressure; and
displaying, on a display interface of a display apparatus, a trend of change over time for the transpulmonary driving pressure, according to transpulmonary driving pressures at different time points.

17. A computer-readable storage medium, **characterized in that**, comprising program(s), which is(are) capable of being executed, so as to implement the method according to any one of claims 11-16.

```
                              ⌒—10              ⌒—20
        ┌──────────┐      ┌──────────┐      ┌──────────┐
        │Respiratory│     │Respirat  │      │          │
        │  assist   │────▶│ory loop  │─────▶│ Patient  │
        │ apparatus │     │          │      │          │
        └──────────┘      └──────────┘      └──────────┘
  30—⌒         │
        ┌──────────┐  │              ┌──────────────┐  ⌒—50
        │ Display  │  │              │First pressure│
        │apparatus │──┤              │   sensor     │
        └──────────┘  │              └──────────────┘
                 ┌─────────┐         ┌──────────────┐  ⌒—60
                 │         │─────────│   Second     │
                 │Processor│         │pressure sensor│
                 │         │         └──────────────┘
                 │         │         ┌──────────────┐  ⌒—70
                 │         │─────────│  Flow sensor │
                 └─────────┘         └──────────────┘
                      ⌒—40
```

FIG. 1

```
             ⌒ 40                        ⌒ 80
                              ┌──────────────────┐
        ┌──────────┐          │  Communication   │
        │          │──────────│    apparatus     │
        │          │          └──────────────────┘
        │Processor │                         ⌒ 30
        │          │          ┌──────────────────┐
        │          │──────────│     Display      │
        └──────────┘          │    apparatus     │
                              └──────────────────┘
```

FIG. 2

Expiratory
branch 213a

213c

214a

Patie
nt

211

Inspiratory
branch 213b

214b

212

Gas

| Memory | Processor | Display |
|---|---|---|

90          40          30

FIG. 3

| Calculate, in real time, end-inspiratory intrapulmonary pressure and end-expiratory intrapulmonary pressure, according to respiratory cycle | 1 |
|---|---|
| Subtract end-expiratory intrapulmonary pressure from end-inspiratory intrapulmonary pressure to obtain real-time driving pressure | 2 |

FIG. 4

Calculate total gas volume of inspiratory phase according to gas flow rate data — 11

Calculate end-inspiratory intrapulmonary pressure according to end-inspiratory airway pressure, total gas volume of inspiratory phase, end-inspiratory flow rate in gas flow rate data, and end-expiratory airway pressure — 12

Calculates end-expiratory intrapulmonary pressure according to end-expiratory airway pressure and end-expiratory flow rate in gas flow rate data — 13

FIG. 5

Calculate a gas volume which corresponds to inspiratory phase, according to gas flow rate which corresponds to inspiratory phase — 11'

Calculate intrapulmonary pressure of inspiratory phase according to airway pressure, gas volume and gas flow rate, which correspond to inspiratory phase — 12'

Calculates intrapulmonary pressure of expiratory phase according to airway pressure and gas flow rate, which correspond to expiratory phase — 13'

Obtain waveform(s) for intrapulmonary pressure according to intrapulmonary pressure of inspiratory phase and intrapulmonary pressure of expiratory phase — 14'

FIG. 6

Respiratory mechanics RC model

FIG. 7

FIG. 8

Obtain airway pressure data and gas flow rate data during
ventilation of respiratory cycle | 1'

Calculate intrapulmonary pressure of inspiratory phase and
intrapulmonary pressure of expiratory phase, according to
airway pressure data and gas flow rate data | 2'

Obtain esophageal pressure data by pressure sensor in
respiratory cycle | 3'

Determine transpulmonary driving pressure of patient which
corresponds to respiratory cycle based on intrapulmonary pressure of
inspiratory phase and intrapulmonary pressure of expiratory phase,
and esophageal pressure measured by pressure sensor during
respiratory cycle | 4'

FIG. 9

## INTERNATIONAL SEARCH REPORT

| International application No. |
|---|
| **PCT/CN2021/141168** |

**A. CLASSIFICATION OF SUBJECT MATTER**

A61M 16/00(2006.01)i

According to International Patent Classification (IPC) or to both national classification and IPC

**B. FIELDS SEARCHED**

Minimum documentation searched (classification system followed by classification symbols)

A61M16

Documentation searched other than minimum documentation to the extent that such documents are included in the fields searched

Electronic data base consulted during the international search (name of data base and, where practicable, search terms used)

CNTXT; CNKI: 吸气末, 峰值, 流量, 正压, 吸气末, 等式, 公式, 呼气末, 峰压, 流速, 压力, 驱动压, 气压, 跨肺, 气道 SIPOABS; ENTXT; DWPI; VEN; SCIENCEDIRECT: LUNG, PEEP, FORMULA, FLOW, PRESSURE, DRIVING PRESSURE, EQUATION, TRANSPULMONARY, INSPIRATORY, EXPIRATORY, ALVEOLAR, AIRWAY

**C. DOCUMENTS CONSIDERED TO BE RELEVANT**

| Category* | Citation of document, with indication, where appropriate, of the relevant passages | Relevant to claim No. |
|---|---|---|
| X | KNOOP, Jennifer L et al. "Model-based estimation of negative inspiratory driving pressure in patients receiving invasive NAVA mechanical ventilation" *Computer Methods and Programs in Biomedicine*, Vol. 208, 22 July 2021 (2021-07-22), abstract, and text, full text | 1, 3-4 |
| Y | KNOOP, Jennifer L et al. "Model-based estimation of negative inspiratory driving pressure in patients receiving invasive NAVA mechanical ventilation" *Computer Methods and Programs in Biomedicine*, Vol. 208, 22 July 2021 (2021-07-22), abstract, and text, full text | 1-17 |
| Y | ARNAL, Jean-Michel et al. "Airway and transpulmonary driving pressures and mechanical powers selected by INTELLiVENT-ASV in passive, mechanically ventilated ICU patients" *Heart & Lung*, Vol. 49, No. 4, 14 November 2019 (2019-11-14), abstract, and text, full text | 1-17 |
| Y | GUY, Ella F. S. et al. "Pilot study of model-based estimation of inspiratory driving pressure in CPAP ventilation" *IFAC-PapersOnline*, Vol. 54, No. 15, 02 November 2021 (2021-11-02), abstract, and text, full text | 1-17 |

☑ Further documents are listed in the continuation of Box C.  ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **05 September 2022** | **16 September 2022** |

| Name and mailing address of the ISA/CN | Authorized officer |
|---|---|
| **China National Intellectual Property Administration (ISA/CN)**<br>**No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

**INTERNATIONAL SEARCH REPORT**

| International application No. |
| --- |
| **PCT/CN2021/141168** |

### C.  DOCUMENTS CONSIDERED TO BE RELEVANT

| Category* | Citation of document, with indication, where appropriate, of the relevant passages | Relevant to claim No. |
| --- | --- | --- |
| Y | WO 2021189197 A1 (SHENZHEN MINDRAY BIO-MEDICAL ELECTRONICS CO., LTD.) 30 September 2021 (2021-09-30) description, page 19, paragraph 3-page 20, paragraph 2, and figure 5 | 1-17 |
| A | CN 108513540 A (THE LUNG BAROMETRY SWEDEN AB) 07 September 2018 (2018-09-07) entire document | 1-17 |
| A | US 2008294060 A1 (CARDIAC PACEMAKERS INC.) 27 November 2008 (2008-11-27) entire document | 1-17 |

Form PCT/ISA/210 (second sheet) (January 2015)

**INTERNATIONAL SEARCH REPORT**
Information on patent family members

International application No.

**PCT/CN2021/141168**

| Patent document cited in search report | | | Publication date (day/month/year) | Patent family member(s) | | | Publication date (day/month/year) |
|---|---|---|---|---|---|---|---|
| WO | 2021189197 | A1 | 30 September 2021 | None | | | |
| CN | 108513540 | A | 07 September 2018 | EP | 3302663 | A1 | 11 April 2018 |
| | | | | SE | 1550671 | A1 | 26 November 2016 |
| | | | | US | 2018140793 | A1 | 24 May 2018 |
| | | | | JP | 2018515292 | A | 14 June 2018 |
| | | | | WO | 2016189069 | A1 | 01 December 2016 |
| | | | | SE | 1550671 | C2 | 10 January 2017 |
| | | | | EP | 3302663 | B1 | 04 September 2019 |
| | | | | JP | 783253 | B2 | 11 November 2020 |
| | | | | US | 10881822 | B2 | 05 January 2021 |
| | | | | CN | 108513540 | B | 23 July 2021 |
| US | 2008294060 | A1 | 27 November 2008 | None | | | |

Form PCT/ISA/210 (patent family annex) (January 2015)